# EUROPEAN PATENT SPECIFICATION

(11) **EP 1 019 731 B1**
(45) Date of publication and mention of the grant of the patent: **08.03.2006**
(21) Application number: 98935606.8
(22) Date of filing: 10.07.1998
(51) Int. Cl.: G01N 33/72, B01J 39/26

(54) **HIGH SPEED CHROMATOGRAPHIC METHOD FOR HEMOGLOBIN VARIANTS SCREENING AND QUANTIFICATION OF HEMOGLOBINS A 2? AND F**
SCHNELLES CHROMATOGRAPHISCHES VERFAHREN ZUR ERFASSUNG VON HÄMOGLOBINVARIANTEN UND ZUR QUANTIFIZIERUNG VON HÄMOGLOBIN A2 UND F
PROCEDE DE CHROMATOGRAPHIE A GRANDE VITESSE PERMETTANT DE DETECTER DES VARIANTS D'HEMOGLOBINE ET DE QUANTIFIER LES HEMOGLOBINES A 2? ET F

(30) Priority: 17.07.1997 US 896037
(43) Date of publication of application: 19.07.2000
(73) Proprietor: PRIMUS CORPORATION, Kansas City, MO 64114 (US)
(72) Inventor: NOFFSINGER, Jimmie, K., Olathe, KS 66062 (US); OU, Ching-Nan, Houston, TX 77071 (US)
(74) Representative: UEXKÜLL & STOLBERG
(86) International application number: PCT/US1998/014363
(87) International publication number: WO 1999/004269

(56) References cited:
- DE-A- 4 124 058
- US-A- 4 108 603
- US-A- 5 292 663
- US-A- 5 417 853
- US-A- 5 719 053
- RIOU JEAN ET AL: "Cation-exchange HPLC evaluated for presumptive identification of hemoglobin variants" CLINICAL CHEMISTRY, vol. 43, no. 1, January 1997 (1997-01), pages 34-39, XP002276791 & ISSN: 0009-9147
- TAN G B ET AL: "Evaluation of high performance liquid chromatography for routine estimation of haemoglobins A-2 and F" JOURNAL OF CLINICAL PATHOLOGY (LONDON), vol. 46, no. 9, 1993, pages 852-856, XP0009029297 & ISSN: 0021-9746
- [Online] XP002276792 Retrieved from the Internet: URL:http://tinyurl.com/yqdcl> [retrieved on 2004-04-13]
- OU CHING-NAN ET AL: "Rapid analysis of hemoglobin variants by cation-exchange HPLC" CLINICAL CHEMISTRY, vol. 39, no. 5, 1993, pages 820-824, XP002276793 & ISSN: 0009-9147
- OU et al., "High-Performance Liquid Chromatography of Human Hemoglobins on a New Cation Exchanger", JOURNAL OF CHROMATOGRAPHY, 1983, Vol. 266, pages 197-205, XP00291253

## Description

### BACKGROUND OF THE INVENTION

### Field of the Invention

The present invention is broadly concerned with improved HPLC methods useful in identifying and characterizing hemoglobin variants in blood. More particularly, the present invention relates to methods in which a blood sample is injected into an HPLC unit and hemoglobin species from the blood sample arc separated using high column temperatures and high eluant flow rates; chromatographic data regarding the separated hemoglobin species are analyzed manually or with aid of a computer to determine if an clured hemoglobin species has a characteristic indicative of a hemoglobin variant and/or to determine if an eluted hemoglobin species corresponds to a known hemoglobin variant; hemoglobin abnonnalities are thereby putatively identified. In particular, the present invention is directed to an improved HPLC assay which provides a relatively low resolution "quick scan" coupled with quantitation of two important hemoglobins, namely A₂ and F; the improved assay is capable of providing such information for respective plural samples with a maximum of 5.5 minutes from injection-to-injection.

### Description of the Prior Art

The hemoglobin molecule is composed of a prosthetic heme group attached to two pairs of unlike polypeptide chains. Normal adult hemoglobin (A) consists of two α and two β chains (α₂β₂). A second normal adult hemoglobin (A₂) consists of two α and two δ chains (α₂δ₂). The primary structure of the δ chain differs from that of the β chain at ten amino acid positions. The blood of normal adult humans contains both A as the major hemoglobin species and A₂ as a minor hemoglobin species. Human fetuses and newborn infants produce mainly fetal hemoglobin (F) which consists of two α chains and two γ chains. The β and γ chains differ at 39 amino acid positions. Additionally, the δ chain and the ε chain have been observed in early human embryos. Newborn infants have two types of γ chain, the ^{G}γ chain which has a glycine residue at position 136, and the ^{A}γ chain which has an alanine residue at position 136. Thus, seven types of hemoglobin chains exist in the human, namely α, β, γ, ^{A}γ, ^{G}γ, δ, and ε chains.

The first abnormal hemoglobin discovered (S) is responsible for sickle cell anemia. S is the result of a substitution of a valine residue for the glutamate residue normally found at position 6 of the β chain. Another relatively common abnormal hemoglobin is C. Most of the hundreds of hemoglobin variants, discovered over the last 40 to 50 years, are the result of single base substitutions in the DNA of either the α, β, ^{A}γ, ^{G}γ, or δ globin genes, but other variants with hybrid chains, extended chains, and chains with deletions have been discovered, The detection of abnormal hemoglobin variants is the primary method of diagnosing hemoglobinopathies.
Approximately 90% of total hemoglobin is nonglycosylated. The major fraction of nonglycosylated hemoglobin is nonglycosylated A, referred to as A₀. The major fraction of glycosylated hemoglobin is glycosylated A, referred to as A_{1c} A_{1c} arises by reaction of a terminal valine amine group in the β chain with the aldehyde group of glucose to give an unstable aldimine. Amadori rearrangement of the aldimine gives A_{1c} which is characterized by a β-ketoglylcoside linked to the valine amine group. The determination of the concentration of A_{1c} is useful in diagnosing diabetes mellitus and in monitoring the treatment of the disease. In nondiabetic individuals, the A_{1c} level is generally between 4 to 8% of total hemoglobin. In diabetics, the A_{1c} level is two- to three-fold higher and may range up to 20% of total hemoglobin.
Numerous procedures have been used to identify and characterize normal and abnormal hemoglobins. Traditionally, these methods have included electrophoresis, isoelectric focusing, and macro-chromatography. During the past several years, high-performance liquid chromatography (HPLC) has been applied to the study of hemoglobinopathies and diabetes mellitus. Advantages of HPLC include relatively high sensitivity, specificity, and speed of analysis. However, HPLC methods generally lack the resolution necessary to differentiate some commonly encountered hemoglobin variants, and also lack the sensitivity required to detect hemoglobins at low concentrations. Additionally, most HPLC methods do not offer computer assisted analysis of the resultant data, and therefore require time-consuming manual analysis.

Riou J et al. (January 1997, Clinical Chemistry 43(1) 34 - 39) describe the presumptive identification of hemoglobin variants using a cation-exchange HPLC on the Bio-Rad Variant automated analyzer with the "β Thalassaemia Short" program.

Tan GB et al. (1993, Journal of Clinical Pathology (London) 46(9) 852 - 856) describe the comparison of HPLC analysis using the Bio-Rad Variant hemoglobin testing system and conventional methods for estimating HbA₂ and HbF concentrations in routine thalassaemia screening.

US 5,292,663 (March 8, 1994) is related to the analysis of blood containing labile glycosylated hemoglobin A_{1c} using an eliminating agent and the Hi-Auto A_{1c} HA-8120 analyzer from Kyoto Daiichi Kagaku.

US 5,417,853 (May 23, 1995) is related to the analysis of blood containing hemoglobin components A₁ₐ, A_{1b}, HbF, A_{1c}, A₀ or A₁ₐ, A_{1b}, HbF, 1-A_{1c}, s-A_{1c}, A₀ respectively using a liquid chromatographic system and eluents of the same composition in both cases.

A high-resolution cation-exchange HPLC method using a polyaspartic acid-containing column was described by Ou and Rognerud in *Clin. Chem.* 39(5):820-824 (1993). Although this method was capable of resolving more than 35 commonly encountered hemoglobin

variants, at least 20 minutes were required for each sample run. Furthermore, this method was not coupled with an automated system of data analysis.
The Primus Corporation of Kansas City, Missouri has heretofore commercialized a two-stage method for the identification of hemoglobin variants in blood samples, which is fully described in application for U.S. Letters Patent, Serial No. 08/642,175 filed May 6.1996. In the Primus assay, a blood sample may be analyzed in a two-step process. In the first step, a "quick scan" HPLC assay is performed at a relatively low resolution in order to identify characteristics indicative of a hemoglobin variant. If such a variant is indicated, the blood sample may be further analyzed by passing a second aliquot thereof through the HPLC unit at a relatively high second resolution in order to provide more reliable quantitative data. As an alternative to a complete quantitative analysis, the Primus assay also permits quantitation of A₂ and F hemoglobins. While the Primus quick scan may be performed with an injection-to-injection time on the order of 2-4 minutes, the higher resolution quantitation step (either the full assay or the A₂/F quantitation) normally takes approximately 10 minutes injection-to-injection.

While the described Primus assay represents a decided break through in the art, users have requested a relatively short-time assay which provides the indicative data of the quick scan together with quantitation of the important A₂ and F hemoglobins.

### SUMMARY OF THE INVENTION

The present invention overcomes the problems outlined above and provides a method for the high speed analysis of a plurality of individual blood samples to determine characteristics therein indicative of a hemoglobin variant, and for quantifying the amounts of A₂ and F hemoglobin in the samples. Broadly speaking, the method of the invention involves analysis of a respective blood sample by passing it through an HPLC column, eluting hemoglobin species with an eluant stream passing through the column, and characterizing the eluted species as a function of their respective adsorbance values and retention times. The characterizing step further includes the step of quantifying the amounts of A₂ and F in the respective blood samples. Importantly, the foregoing analysis of the respective blood sample is repeated in order to analyze each of the plurality of samples, wherein the total time between successive analysis steps is up to about. 5.5 minutes. The time between successive analysis steps is generally referred to as the injection-to-injection time at the HPLC column.

In carrying out the method of the invention, it is preferred to maintain the HPLC column at a level of from 30-45°C and more preferably about 40°C. The HPLC column is preferably a cation exchange column and especially a polyaspartic acid column including a porous silica support. Column pressures in the method are dependent variables, i.e., the pressures result from factors such as column packing particle size, flow rates, and temperatures. Generally speaking, pressure drops across the column of from 2,41x10⁶⁻10,34x10⁶Pa (350-1500 psi) are observed.

In preferred forms, two mobile phases are employed in the analysis (i.e., mobile phase 1 and mobile phase 2). The flow rates of the eluants through the column are normally from 2-5 ml/min., and more typically from 2-3 ml/min. In order to obtain the desired short time high speed analyses of plural samples, it is desirable to establish a non-linear mobile phase gradient between mobile phase 1 and mobile phase 2. That is to say, a non-linear mobile phase gradient covering a range of concentrations from 70-95% mobile phase 1 and 5-30% mobile phase 2 up to 100% mobile phase 2 over a period of from 2-5 minutes is employed to facilitate separation of hemoglobin components. The most preferred gradient covers a range of from about 90% mobile phase 1/10% mobile phase 2 to 100% mobile phase 2 over a period of from 4-5 minutes.

Generally speaking, the mobile phase I has a relatively low total ionic strength and an approximately neutral pH. This phase should contain from 5-50 meq of cation and a pH of from 6.8-7.5. Mobile phase 2 should have a significantly higher total ionic strength and typically a somewhat lower pH. For example, this phase would normally have from 100-300 meq of cation and a pH of from 6-6.9. By appropriate manipulation of the relative pHs and ionic strengths of the mobile phases, it is possible to separate hemoglobin fractions with very similar physical properties.

The improved method described herein is similar in some respects to the methods described in pending application for U.S. Letters Patent, Serial No. 08/642,175 filed May 6, 1996.

### BRIEF DESCRIPTION OF THE DRAWINGS

Figure 1 is an individual sample report generated using the quick scan/A₂ and F quantitation assay described in Example 1; and
Fig. 2 is an individual sample report generated using the quick scan/A₂ and F quantitation assay described in Example 2.

### DETAILED DESCRIPTION OF THE PREFERRED EMBODIMENT

The present invention is directed to an HPLC chromatographic method for the identification and characterization of hemoglobin variants in blood samples from patients. In particular, the invention is directed to a combined assay giving "quick scan" hemoglobin variant indicative information together with quantitation of the important A₂ and F hemoglobins.

The following examples set forth preferred techniques in accordance with the invention. It is to be understood however, that these examples are presented by way of illustration only and nothing therein should be taken as a limitation upon the overall scope of the invention.

### Example 1

Instrumentation. The HPLC unit used in the chromatographic analysis of hemoglobin was the continuous, automated Hewlett Packard 1090 as modified by Primus Corporation and known as the Primus CLC330. This unit included a proportioning valve, a metering pump, a high pressure booster pump, an auto sampler, an auto injector, a photometric detector, dual disc drives, a recording integrator, a temperature-controlled oven, and an HP LUCI controller. However, the analysis can be conducted with any high-quality HPLC system using the appropriate parameters.

Chemistry. Separation of hemoglobin components took place on a cation exchange column. Specifically, a polyaspartic acid column (3.5 cm x 0.46 cm) including a porous silica support having a 5 µm particle size and a pore size of 100 nm was used, although many other types of cation exchange columns can be employed. Two mobile phases were used in the HPLC unit. Mobile phase 1 was composed of 10 mM Bis-Tris and 1 mM KCN, and had a pH of 7.0; this phase therefore has a total ionic strength of I 1 meq of cation. Mobile phase 2 was composed of 10 mM Bis-Tris,1 mM KCN, and 200 mM NaCl, and had a pH of 6.6; this phase had a total ionic strength of 211 meq of cation. The amounts of mobile phase 1 and mobile phase 2 in a mixture thereof are represented below as volume percentages relative to the total volume of the mixture. The test samples contained whole blood hemolysate prepared by dilution of one part whole blood with from about 20 to 500 parts purified water (preferably from 50 to 200 parts purified water, and ideally 100 parts purified water). Sodium azide (0.001%) was added as a preservative. The injection volume was from about 1 µl to 30 µl (preferably from 2.5 µl to 20 µl, and ideally 5 µl) of the diluted (i.e., hemolyzed) sample.

During startup, the lamp and pumps for the unit were turned on. The flow rate of eluant was set to 2.5 ml/minute, and the column temperature was set to 40°C. Initially, 100% mobile phase 2 was pumped through the system for 4.9 minutes (i.e., from time 0 to time 4.9 minutes). From time 4.9 minutes to time 5.0 minutes, the composition of the eluant was changed in a linear fashion from 100% mobile phase 2 to 90% mobile phase 1 and 10% mobile phase 2; this condition was held constant from time 5.0 minutes to time 10.0 minutes. From time 10.0 minutes to time 13.0 minutes, the composition of the eluant was changed in a linear fashion from 90% mobile phase 1 and 10% mobile phase 2 to 60% mobile phase 1 and 40% mobile phase 2. From time 13.0 minutes to time 16.0 minutes, the composition of the eluant was changed in a linear fashion from 60% mobile phase 1 and 40% mobile phase 2 to 100% mobile phase 2. At time 16.0, the unit was ready for the quick scan/A₂ and F quantitation assay.

The HPLC column was next equilibrated over a period of 1.4 minutes with a mixture of 85% mobile phase 1 and 15% mobile phase 2, at a constant flow rate of 3.0 ml/minute for 0.4 minute followed by 1.4 minutes at a constant flow rate of 2.5 ml/minute. Throughout the analysis, i.e., during equilibration and thereafter, the column temperature was maintained at 40°C and the eluant employed was passed through the column at a flow rate beginning at 2.5 ml/minute and increasing linearly to 3.0 ml/minute over a period of 2 minutes. The flow was then kept constant until 4.1 minutes, at which time it was reduced immediately to 2.5 ml/minute. A non-linear mobile phase gradient covering a range of concentrations from 90% mobile phase 1 and 10% mobile phase 2 to 100% mobile phase 2 was used to separate hemoglobin components. The gradient changes took place over 4 minutes. Thereafter, the conditions were returned to the starting conditions over a period of 0.1 minute. The non-linear mobile phase gradient included the following steps. At time of injection (i.e., time zero), the eluant consisted of 90% mobile phase 1 and 10% mobile phase 2. Over the first minute (i.e., to time 1.0 minute), the composition of the eluant was changed in a linear fashion to consist of 86% mobile phase 1 and 14% mobile phase 2. From time 1.0 minute until time 2.2 minutes, the eluant was changed in a linear fashion to consist of 70% mobile phase 1 and 30% mobile phase 2. From time 2.2 minutes to time 2.8, the eluant was changed in a linear fashion to consist of 50% mobile phase 1 and 50% mobile phase 2. From time 2.8 minutes until time 3.2 minutes, the eluant was changed in a linear fashion to consist of 75% mobile phase 1 and 25% mobile phase 2. From time 3.2 minutes until 3.21 minutes, the eluant was changed in a linear fashion to consist of 100% mobile phase 2. This eluant condition was then maintained for an additional 0.79 minutes (i.e., until time 4.0 minutes). From time 4.0 minutes until time 4.1 minutes, the eluant was changed in a linear fashion to consist of 90% mobile phase I and 10% mobile phase 2. This eluant condition was maintained for an additional 1.4 minutes (i.e., until time 5.5 minutes). Thus, a total of 5.5 minutes elapsed from injection to injection.

The first two samples of the batch run were respective aliquots of a known marker sample containing F, A, A₂, S, and C. However, any other hemoglobin sample could also be used in lieu of the marker sample. Results of this first injection were ignored. The second sample injected was deemed to be the marker sample. The sequential order of elution of the hemoglobin species in the marker sample is known to be F. A, A₂, S, and C (the largest peak between the A and S peaks is known to be the A₂ peak). The resultant chromatograph was used to calculate the absolute retention time, area percent value, and peak width for each of F, A, A₂, S, and C. Area percent value refers to the percentage of a particular species' integrated peak area relative to the total integrated peak area of the entire chromatograph. Marker samples were used as described above to recalibrate the HPLC unit with respect to absolute retention times, area percent values, and peak width usually after 20 unknown sample runs. However, recalibration was necessary only after 100 sample runs.

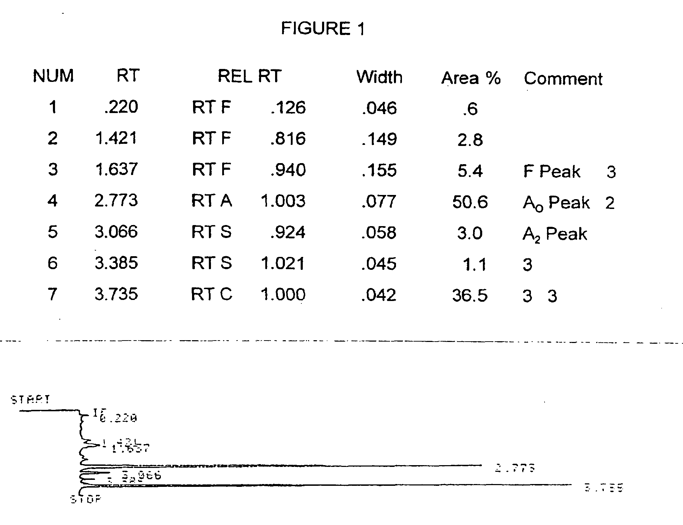

Fig. 1 illustrates a typical chromatograph report for a respective sample obtained using the described techniques, together with the computer-generated numerical data. As shown, each report includes a chromatograph of the sample and a table including the number of each peak, the absolute retention time of the species giving the peak, the closest marker peak (i.e., F, A, S or C), the relative retention time of the species giving the peak (i.e., the absolute retention time of the species giving the peak divided by the absolute retention time of the closest marker species), the width of the peak, the area percent value of the peak and comments. Code numbers are included in the comment section where appropriate. Also, peaks may be putatively identified in the comments section by determining which species had a relative retention time falling within an individual window (see Table 3). The illustrated chromatograph is one where significant F and A₂ peaks were observed. It will be understood however that many samples will not contain significant F peaks.

The rules shown in Table 1 were used to identify abnormal hemoglobin species in blood samples.

**Table 1. Rules used to identify abnormal hemoglobin patterns in Quick Scan/A, and F quantitation chromatographs.**

| Rule | Description |
|---|---|
| 1 | Any peak (other than the A₀ or A_{1c} peaks) having an area percent value greater than 5% is flagged (code 3).' |
| 2 | Any peak in the A_{1c} window having an area percent value greater than 10% is flagged (code 3).^{2.3} |
| 3 | Any peak in the A₀ window having an area percent value less than 80% is flagged (code 2).⁴ |
| 4 | Any peak in the A₀ window having a width greater than 1.2 times the width of the marker A₀ peak is flagged (code 1).⁵ |
| 5 | Any peak in the degradation products window having an area percent value greater than 10% is flagged (code 3).⁶ |
| 6 | Any peak eluting after the A₂ window is flagged (code 3). |
| 7 | Any peak in the hemoglobin Bart's window having an area percent value greater than 1% is flagged (code 3). |
| 8 | If any peak in the A₂ window has an area percent value greater than 10%, the result is printed as not valid for A₂ quantitation and thus signals the presence of a variant comigrating with A₂. |

| | |
|---|---|
| ¹See Table 2 for Codes. | |
| ²See Table 3 for windows for A_{1c}, A₀, degradation products, A₂, and hemoglobin Bart's. | |
| ³The A_{1c} peak has an area percent value greater than 10% of total hemoglobin only when an abnormal hemoglobin is present or in poorly controlled diabetes. | |
| ⁴The A₀ peak has an area percent value always greater than 80% of total hemoglobin except in cases of thalassemia, abnormal hemoglobin, or in very poorly controlled diabetes. | |
| ⁵When A₀ and hemoglobin variants eluting very close to A₀ were both present in the same sample, the two hemoglobins blended together as a single peak which was shorter and broader than a peak composed of a single substance. | |
| ⁶Degradation products result from hemoglobin degradation in older samples. | |

**Table 2. Codes.**

| Code | Description |
|---|---|
| 1 | The width of the A₀ peak is greater than 1.2 times the width of the marker A₀ peak. |
| 2 | The area percent value of the A₀ peak is less than 80%. |
| 3 | The area percent value of any peak other than the A₀ peak is greater than the expected area percent value for that peak. |

**Table 3. Windows for A_{1c}, A₀, degradation products, A₂, hemoglobin Bart's, and F.**

| Hemoglobin | Window¹ |
|---|---|
| A_{1c} | 0.6-0.9² |
| A₀ | 0.98-1.03³ |
| Degradation Products | 0.75-0.89⁴ |
| A₂ | 1.05-1.20⁵ |
| Bart's | < 0.18⁶ |
| F | 0.90-1.05⁷ |
| Acetylated F | 0.28-0.40⁸ |

| | |
|---|---|
| ¹Windows are expressed either as a range of values for either absolute retention time or relative retention time; relative retention time is determined by dividing the absolute retention time of one species by the absolute retention time of another species; the window for each particular species noted in Table 3 was determined experimentally by running one or more samples known to contain that species. | |
| ²Relative retention time (absolute retention time of known A_{1c} divided by the absolute retention time of marker F). | |
| ³Relative retention time (absolute retention time of known A₀ divided by the absolute retention time of marker A). | |
| ⁴Relative retention time (absolute retention time of known degradation products divided by the absolute retention time of marker A). | |
| ⁵Relative retention time (absolute retention time of known A₂ divided by the absolute retention time of marker A). | |
| ⁶Absolute retention time in minutes. | |
| ⁷Relative retention time (absolute retention time of known F divided by the absolute retention time of marker F, with known F and marker F being in different samples). | |
| ⁸Relative retention time (absolute retention time of known Acetylated F divided by the absolute retention time of marker F). | |

At the conclusion of each batch run, a batch summary report is printed. Each batch summary report lists every sample which has been flagged as abnormal. The batch sample report includes the following information: a) the sample number, b) the number of the peak which was abnormal, c) the absolute retention time of the species giving the peak, d) the relative retention time of the species giving the peak (i.e., the absolute retention time of the species giving the peak divided by the absolute retention time of the closest marker species), e) the width of the peak, f) the area percent value of the peak, g) the quantitative A₂ and F values, and h) one or more of the code numbers described above.

The data generated in this example was highly reproducible and accurate. A statistical analysis of the data indicated that the correlation coefficient was greater than 0.99 when compared with the 10 minute high resolution method.

### Example 2

In this example, a series of quick scan/A₂ and F quantitation assays were performed as described above using a different lot ofHPLC columns, which were found to have significantly different properties. This necessitated a change in both reagent composition and the non-linear eluant gradients employed. In particular, mobile phase 1 was composed of 20 mM Bis-Tris and 2 mM KCN, and had a pH of 7.0. Mobile phase 2 was composed of 20 mM Bis-Tris, 2 mM KCN and 200 mM NaCl and had a pH of 6.6.

After start-up was completed, the column was equilibrated over 2 minutes with a mixture of 85% mobile phase 1 and 15% mobile phase 2 at a constant flow rate of 3.0 ml/minute for 0.5 minutes followed by 1.5 minutes at 2.5 ml/minute. Throughout the analysis (i.e., during equilibration and thereafter), the column temperature was maintained at 40°C, and the eluant employed was passed through the column at a constant flow rate of 2.5 ml/minute for the first 2.3 minutes and increasing linearly to 3.0 ml/minute over a period of 0.5 minutes (i.e., from 2.3 minutes to 2.8 minutes). The flow was then kept constant until 4.0 minutes at which time it was reduced immediately to 2.5 ml/minute. A non-linear mobile-phase gradient covering a range of concentrations from 90% mobile phase I and 10% mobile phase 2 to 100% mobile phase 2 was used to separate hemoglobin components. The gradient changes took place over 3.5 minutes. Thereafter, the conditions were returned to the starting conditions over 0.1 minute.

The non-linear mobile phase gradient included the following steps. At time of injection (i.e., time zero), the eluant consisted of 90% mobile phase I and 10% mobile phase 2. Over the first 0.4 minutes (i.e., to time 0.4 minute), the composition of the eluant was changed in a linear fashion to consist of 86% mobile phase I and 14% mobile phase 2. From time 0.4 minutes until time 1.0 minutes, the eluant was kept constant at 86% mobile phase I and 14% mobile phase 2. From time 1.0 minutes to time 2.3 the eluant was changed in a linear fashion to consist of 70% mobile phase I and 30% mobile phase 2. From time 2.3 minutes until time 2.8 minutes the eluant was changed in a linear fashion to consist of 50% mobile phase I and mobile phase 2. From time 2.8 minutes until 3.2 minutes the eluant was changed in a linear fashion to consist of 25% mobile phase 1 and 75% mobile phase 2. From time 3.2 minutes until time 3.21 minutes the eluant was changed in a linear fashion to consist of 100% mobile phase 2. This eluant condition was then maintained for an additional 0.39 minutes (i.e., until time 3.5 minutes). From time 3.5 minutes until time 3.51 minutes the eluant was changed in a linear fashion to consist of 85% mobile phase I and 15% mobile phase 2. This eluant condition was maintained for an additional 1.99 minutes (i.e., until time 5.5 minutes). Thus, a total time of 5.5 minutes elapsed from injection to injection.

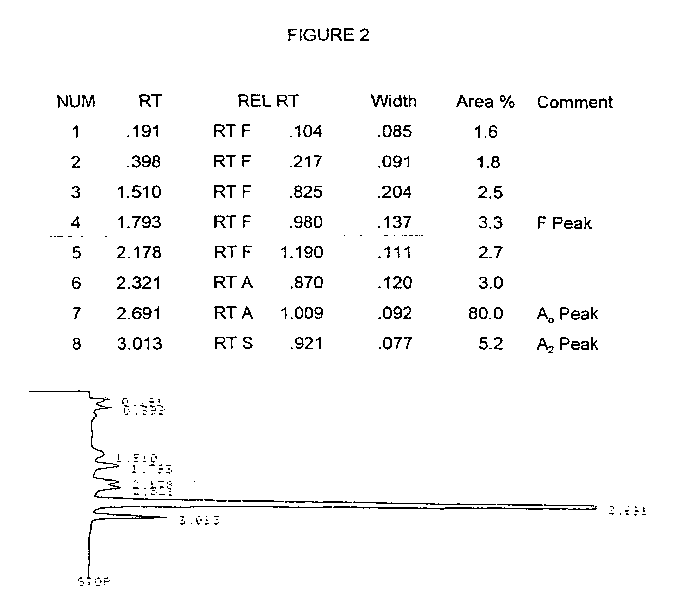

Fig. 2 is a representative output report from one of the samples tested in this batch.

### Computer Assistance

The above-described steps and calculations may be performed manually. However, they are preferably performed with the aid of the computer program. The computer program operates a conventional microcomputer, minicomputer, or mainframe computer and is stored on a conventional computer-readable memory device such as a floppy or optical disk of the hard drive of the computer.

## Claims

1. A method for analyzing a plurality of individual blood samples to determine characteristics therein indicative of a hemoglobin variant, and for quantifying the amounts of A₂ and F hemoglobin in said samples, said method comprising the steps of:
(a) analyzing a respective blood sample by passing it through an HPLC column, said HPLC column consisting essentially of a cation exchange column maintained above about 30° C;
(b) eluting normal and abnormal hemoglobin species including Hb-C with an eluant stream passing through the HPLC column;
(c) characterizing eluted species as a function of their respective absorbance values and retention times, said characterizing step including the step, of quantifying the amounts of A₂ and F hemoglobins in the respective blood sample; and
(d) repeating steps (a-c) to analyze each of the plurality of blood samples, wherein the total time between successive steps (a-c) is up to about 5.5 minutes.

2. The method of claim 1, including the step of Maintaining said column temperature at a level of from 30-45° C.

3. The method of claim 2, said temperature being about 40° C.

4. The method of claim 1, including the step of passing said eluant through said column at a flow rate of from 2-5 ml/min.

5. A method for analyzing a plurality of individual blood samples to determine characteristics therein indicative of a hemoglobin variant, and for quantifying the amounts of A₂ and F hemoglobin in said samples, said method comprising the steps of:
(a) analyzing a respective blood sample by passing it through an HPLC column, said HPLC column comprising a polyaspartic acid column maintained above about 30° C;
(b) eluting hemoglobin species with an eluant stream passing through said HPLC column;
(c) characterizing eluted species as a function of their respective absorbance values and retention times, said characterizing step including a step of quantifying the amounts of A₂ and F hemoglobins in the respective blood samples; and
(d) repeating steps (a-c) to analyze each of the plurality of blood samples, wherein the total time between successive steps (a-c) including column reequilibriation is up to about 5.5 minutes.

## Patentansprüche

1. Verfahren zur Analyse von Eigenschaften einer Vielzahl individueller Blutproben, wobei die Eigenschaften auf eine Hämoglobin-Variante hinweisen, und zur Quantifizierung der Mengen von A₂ und F Hämoglobin in den Proben, Schritte umfassend, bei denen man:
(a) eine entsprechende Blutprobe analysiert, indem diese über eine HPLC-Säule geleitet wird, wobei die besagte HPLC-Säule im wesentlichen aus einer Kationenaustauschersäule besteht, die oberhalb von etwa 30°C gehalten wird;
(b) normale und nicht-normale Hämoglobinarten inklusive Hb-C mit einem Elutionsstrom eluiert, der durch die HPLC-Säule geleitet wird;
(c) die Eigenschaften der eluierten Arten in Abhängigkeit der jeweiligen Absorptionswerte und Retentionszeiten bestimmt, wobei der Schritt der Bestimmung der Eigenschaften die Quantifizierung der Mengen des A2- und F-Hämoglobins in den entsprechenden Blutprobe umfaßt; und
(d) die Schritte (a-c) wiederholt, um jede der Vielzahl der Blutproben zu analysieren, wobei die Gesamtzeit zwischen den aufeinanderfolgenden Schritten (a-c) bis zu ungefähr 5.5 Minuten beträgt.

2. Verfahren gemäß Anspruch 1, bei dem man die Temperatur der Säule auf einem Bereich von 30-45°C beläßt.

3. Verfahren gemäß Anspruch 2, bei dem die Temperatur etwa 40°C beträgt.

4. Verfahren gemäß Anspruch 1, bei dem man das Elutionsmittel durch die Säule in einer Flußrate von 2-5 ml/min laufen läßt.

5. Verfahren zur Analyse von Eigenschaften in einer Vielzahl individueller Blutproben, wobei die Eigenschaften auf eine Hämoglobin-Variante hinweisen, und zur Quantifizierung der Mengen von A₂- und F-Hämoglobin in den Proben, Schritte umfassend, bei denen man:
(a) eine entsprechende Blutprobe analysiert, indem diese über eine HPLC-Säule geleitet wird, wobei die besagte HPLC-Säule eine Polyaspartat, die oberhalb von etwa 30°C gehalten wird;
(b) die Hämoglobinarten mit einem Elutionsstrom eluiert, der durch die-HPLC Säule geleitet wird;
(c) die Eigenschaften der eluierten Arten in Abhängigkeit der jeweiligen Absorptionswerte und Retentionszeiten bestimmt, wobei der Schritt der Bestimmung der Eigenschaften die Quantifizierung der Mengen des A2- und F-Hämoglobins in den entsprechenden Blutprobe umfaßt; und
(d) die Schritte (a-c) wiederholt, um jede der Vielzahl der Blutproben zu analysieren, wobei die Gesamtzeit zwischen den aufeinanderfolgenden Schritten (a-c) inklusive der Reequilibrierung der Säule bis zu ungefähr 5.5 Minuten beträgt.

## Revendications

1. Procédé pour analyser une pluralité d'échantillons sanguins individuels pour en déterminer les caractéristiques indiquant un variant d'hémoglobine et pour quantifier les quantités d'hémoglobines A₂ et F dans lesdits échantillons, ledit procédé comprenant les étapes suivantes :
(a) l'analyse d'un échantillon sanguin respectif en le faisant passer à travers une colonne de HPLC, ladite colonne de HPLC étant essentiellement constituée d'une colonne échangeuse de cations maintenue au-dessus d'environ 30°C;
(b) l'élution d'espèces normales et anormales d'hémoglobines, incluant la Hb-C avec un courant d'éluant passant à travers la colonne de HPLC;
(c) la caractérisation des espèces éluées en fonction de leurs valeurs d'absorbance et temps de rétention respectifs, ladite étape de caractérisation comprenant l'étape de quantification des quantités d'hémoglobines A₂ et F dans l'échantillon sanguin respectif; et
(d) la répétition des étapes (a-c) pour analyser chacun de la pluralité d'échantillons sanguins, dans laquelle la durée totale entre les étapes successives (a-c) s'étendant jusqu'à environ 5,5 minutes.

2. Procédé selon la revendication 1, comprenant l'étape de maintien de ladite température de la colonne à un niveau de 30 à 45°C.

3. Procédé selon la revendication 2, dans lequel ladite température est d'environ 40°C.

4. Procédé selon la revendication 1, comprenant l'étape consistant à faire passer ledit éluant à travers ladite colonne avec un débit d'environ 2 à 5 ml/mn.

5. Procédé pour analyser une pluralité d'échantillons sanguins individuels pour en déterminer les caractéristiques indiquant un variant d'hémoglobine et pour quantifier les quantités d'hémoglobines A₂ et F dans lesdits échantillons, ledit procédé comprenant les étapes suivantes :
(a) l'analyse d'un échantillon sanguin respectif en le faisant passer à travers une colonne de HPLC, ladite colonne de HPLC comprenant une colonne d'acide polyaspartique maintenue au-dessus d'environ 30°C;
(b) l'élution des espèces d'hémoglobines avec un courant d'éluant passant à travers ladite colonne de HPLC;
(c) la caractérisation des espèces éluées en fonction de leurs valeurs d'absorbance et temps de rétention respectifs, ladite étape de caractérisation comprenant l'étape de quantification des quantités d'hémoglobines A₂ et F dans l'échantillon sanguin respectif; et
(d) la répétition des étapes (a-c) pour analyser chacun de la pluralité d'échantillons sanguins, dans laquelle la durée totale entre les étapes successives (a-c), y compris le rééquilibrage de la colonne, s'étendant jusqu'à environ 5,5 minutes.
